# EUROPÄISCHE PATENTANMELDUNG

(11) **EP 4 484 410 A1**
(43) Veröffentlichungstag der Anmeldung: **01.01.2025**
(21) Anmeldenummer: 23182060.6
(22) Anmeldetag: 28.06.2023
(51) Int. Cl.: C07D 209/82

(54) **VERFAHREN ZUR HERSTELLUNG VON N-ALKYLCARBAZOLEN DURCH METALLFREIE AROMATISIERUNG**

(71) Anmelder: Heubach Holding Switzerland Ltd, 4133 Pratteln (CH)
(72) Erfinder: ENDRES, Andreas, 65926 Frankfurt am Main (DE); HAUCK, Stefan, 65926 Frankfurt am Main (DE)
(74) Vertreter: Schuck, Alexander

(57) **Zusammenfassung**

Verfahren zur Herstellung von N-Alkylcarbazolen der allgemeinen Formel 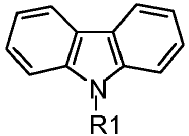 worin R₁ ein linearer oder verzweigter C₁-C₁₂-Alkylrest ist, durch metallfreie Aromatisierung eines teilhydrierten oder perhydrierten N-Alkylcarbazols in Gegenwart von Aktivkohle und Sauerstoff, dadurch gekennzeichnet, dass das teilhydrierte oder perhydrierte N-Alkylcarbazol in Gegenwart von Aktivkohle unter einer sauerstoffhaltigen Atmosphäre mit einem Sauerstoffgehalt von 10 bis 30 Vol.-% auf eine Temperatur von 150 bis 300°C erhitzt wird.

## Beschreibung

Die Erfindung betrifft ein Verfahren zur Herstellung von N-Alkylcarbazolen der allgemeinen Formel 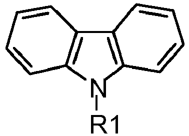 worin R₁ ein linearer oder verzweigter C₁-C₁₂-Alkylrest ist, durch metallfreie Aromatisierung eines teilhydrierten oder perhydrierten N-Alkylcarbazols.

N-Alkylcarbazole sind wichtige Zwischenprodukte, z.B. für die Herstellung von Pigment Violett 23 (hier ist Alkyl = Ethyl), darüber hinaus werden N-Alkylcarbazole für Anwendungen in der molekularen Elektronik, den Materialwissenschaften und in der Herstellung von Dispersionsfarbstoffen benötigt. In jüngerer Zeit wurden perhydrierte Derivate von N-Alkylcarbazolen als flüssiger organischer Wasserstoffspeicher für elektrolytisch erzeugten Wasserstoff vorgeschlagen, siehe Papp et al., Nachrichten aus der Chemie 62 (2014), S. 965.

Insbesondere das N-Ethylcarbazol ist ein wichtiges Zwischenprodukt, das für die Herstellung des wichtigen Pigments PV 23 benötigt wird. Eine gebräuchliche Methode zur Herstellung von N-Alkylcarbazolen beinhaltet die Alkylierung von aus Steinkohleteer gewonnenem Carbazol mit Alkylierungsmitteln wie Dialkylsulfat, Dialkylcarbonat oder Alkylhalogeniden in Gegenwart von Basen. Nachteilig an dieser Methode ist die schwierige vollständige N-Alkylierung von Carbazol sowie die oft nicht ausreichende Reinheit des erhaltenen Produkts, was auch an den im Carbazol enthaltenen Nebenprodukten, z.B. Acridin, liegt.

N-Alkylcarbazole sind auch durch Aromatisierung der entsprechenden N-Alkyltetrahydrocarbazole herstellbar. Letztere sind leicht mittels einer Fischer-Indolsynthese aus Cyclohexanon und dem entsprechenden N-Alkyl-N-phenylhydrazin herstellbar, wie beschrieben in Fletcher, J. Chem. Soc. (1953), S. 3898. Das N-Ethyl-N-phenylhydrazin wurde bereits im 19. Jahrhundert synthetisiert, wie beschrieben in E. Fischer, Chem. Ber. 8 (1875), S. 1641.

E. C. Horning et. al., J. Am. Chem. Soc. 70 (1948), S. 3935, beschreiben die Palladiumkatalysierte Herstellung von N-Alkylcarbazolen aus den entsprechenden N-Alkyl-1,2,3,4-tetrahydrocarbazolen. Nachteile dieser Methode sind der hohe Palladiumpreis und der teilweise Verlust des Metalls durch Austrag ("Leaching") sowie die Deaktivierung des Katalysators durch Ablagerung von Nebenprodukten auf dessen Oberfläche.

Die V₂O₅-katalysierte Aromatisierung von nicht am Stickstoff substituierten 1,2,3,4-Tetrahydrocarbazolen, wie beschrieben in Magolan et. al., Synlett 2013. S. 1675, ist auch auf deren N-Alkylderivate anwendbar, nachteilig ist allerdings die sehr lange Reaktionszeit bei erhöhten Temperaturen und die schwierige Entfernung des giftigen Vanadium-Katalysators.

Die seit langem bekannten Aromatisierungen mit 2,3-Dichloro-5,6-dicyano-1,4-benzoquinon (DDQ), Chloranil oder Schwefel sind auch bei N-Alkyl-1,2,3,4-tetrahydrocarbazolen anwendbar, erfordern allerdings die Entfernung der reduzierten p-Chinone bzw. sind mit der Bildung von giftigem Schwefelwasserstoff verbunden. Die lod-Aromatisierung von nicht am Stickstoff substituierten 1,2,3,4-Tetrahydrocarbazolen, wie beschrieben in P. D. Lokande et. al., J. Org. Chem. 80 (2015), S. 2392, benötigt größere Mengen (bis zu 50 mol-%) elementares Iod sowie DMSO als Lösungsmittel bzw. Co-Oxidans, und ist für den industriellen Maßstab weniger geeignet. Zudem kann eine Iodierung des Carbazols auftreten. Weiterhin waren die in eigenen Versuchen erzielten Ausbeuten bei der Verwendung von N-Alkyl-1,2,3,4-tetrahydrocarbazolen niedrig.

P. D. Lokande et. al., Tetrahedron Lett. 59 (2018), S. 2145, beschreiben auch die CuCl₂/DMSO-katalysierte Aromatisierung von N-unsubstituierten 1,2,3,4-Tetrahydrocarbazolen. Neben dem toxikologisch bedenklichen Kupfer und dem DMSO waren die in eigenen Versuchen zur Aromatisierung von N-Alkyl-1,2,3,4-tetrahydrocarbazolen erzielten Ausbeuten gering.

CN102633710A beschreibt die Dehydrierung von 1,2,3,4-Tetrahydrocarbazol-Verbindungen zu den entsprechenden Carbazol-Verbindungen in zwei Stufen, wobei in der ersten Stufe ein kostengünstiger Katalysator mit niedriger Aktivität und in der zweiten Stufe ein Edelmetallkatalysator mit hoher Aktivität eingesetzt werden, oder umgekehrt. Als kostengünstige Katalysatoren mit niedriger Aktivität werden Aktivkohle, Diatomeenerde, Siliziumdioxid und Raney-Nickel genannt. Als Edelmetallkatalysatoren mit hoher Aktivität werden Palladium, Platin und Ruthenium auf Aktivkohle, Aluminiumoxid oder mesoporösem Kohlenstoff genannt. In den Ausführungsbeispielen werden Tetrahydrocarbazol, N-Methyltetrahydrocarbazol und N-Ethyltetrahydrocarbazol zu den entsprechenden Carbazolen dehydriert. Dabei wird in einigen Beispielen Aktivkohle als kostengünstiger Katalysator mit Palladium auf mesoporösem Kohlenstoff kombiniert.

Dong Yuan et al.: "Fast Dehydrogenation Kinetics of Perhydro-N-propylcarbazole over a Supported Pd Catalyst", ACS Applied Energy Materials 2018, Bd. 1, 4285-4292 (D2) offenbart eine kinetische Untersuchung der Dehydrierung von Perhydro-N-propylcarbazol an einem geträgerten Pd-Katalysator im Temperaturbereich von 160-200°C bei Atmosphärendruck. Es wird berichtet, dass die Dehydrierung in drei aufeinanderfolgenden Stufen über Octahydro-N-propylcarbazol, Tetrahydro-N-propylcarbazol zu N-Propylcarbazol erfolgt.

T. Tanaka et al., Tetrahedron Letter 51 (2010), 4633 - 4635 berichten die Dehydrierung von 1,2,3,4-Tetrahydrocarbazol zu Carbazol an Aktivkohle in einer Sauerstoff-Atmosphäre bei 120°C in Xylol als Lösungsmittel. Das Arbeiten in Xylol in Gegenwart von reinem Sauerstoff ist sicherheitstechnisch sehr bedenklich.

Somit sind bei der Herstellung von N-Alkylcarbazolen weitere Verbesserungen und Vereinfachungen des Aromatisierungsschrittes wünschenswert. Aufgabe der Erfindung ist es daher, ein einfacheres Verfahren zur Herstellung von N-Alkylcarbazolen umfassend die Aromatisierung von hydrierten N-Alkylcarbazols bereitzustellen. Aufgabe der Erfindung ist es insbesondere, ein solches Verfahren bereitzustellen, das ohne edelmetallhaltige Katalysatoren auskommt.

Gelöst wird die Aufgabe durch ein Verfahren zur Herstellung von N-Alkylcarbazolen der allgemeinen Formel 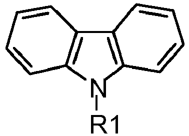 worin R₁ ein linearer oder verzweigter C₁-C₁₂-Alkylrest ist, durch metallfreie Aromatisierung eines teilhydrierten oder perhydrierten N-Alkylcarbazols in Gegenwart von Aktivkohle und Sauerstoff, dadurch gekennzeichnet, dass das teilhydrierte oder perhydrierte N-Alkylcarbazol in Gegenwart von Aktivkohle unter einer sauerstoffhaltigen Atmosphäre mit einem Sauerstoffgehalt von 10 bis 30 Vol.-% auf eine Temperatur von 150 bis 300°C erhitzt wird.

Durch das Arbeiten unter einer sauerstoffhaltigen Atmosphäre mit einem Sauerstoffgehalt von nur 10 bis 30 Vol.-% wird eine sicherere Fahrweise realisiert.

Geeignete teilhydrierte N-Alkylcarbazole sind die isomeren N-Alkyltetrahydrocarbazole, N-Alkylhexahydrocarbazole, N-Alkyloctahydrocarbazole und N-Alkyldecahydrocarbazole. Perhydrierte N-Alkylcarbazole sind N-Alkyldodecahydrocarbazole. Bevorzugte teilhydrierte N-Alkylcarbazole sind N-Alkyl-1,2,3,4,5,6,7,8-octahydrocarbazole, N-Alkyl-1,2,3,4,4a,9a-hexahydrocarbazole und N-Alkyl-1,2,3,4-tetrahydrocarbazole.

Besonders bevorzugte teilhydrierte N-Alkylcarbazole sind N-Alkyl-1,2,3,4-tetrahydro-carbazole.

N-Alkyl-1,2,3,4-tetrahydrocarbazole können durch Fischer-Indolsynthese aus Cyclohexanon und dem entsprechenden N-Alkyl-N-phenylhydrazin hergestellt werden.

R₁ ist bevorzugt ein linearer oder verzweigter C₁-C₈-Alkylrest, insbesondere ist R₁ ein Ethylrest.

Eine speziell bevorzugte Verbindung ist N-Ethyl-1,2,3,4-tetrahydrocarbazol. Diese kann durch Fischer-Indolsynthese aus Cyclohexanon und dem N-Ethyl-N-phenylhydrazin hergestellt werden.

In dem erfindungsgemäßen Verfahren wird die Aromatisierung ohne oder in Gegenwart eines im allgemeinen hochsiedenden organischen Lösungsmittels, z.B. Diethylenglycol, Triethylenglycol, Diglyme, Triglyme, N-Methylpyrrolidon, Ethylencarbonat, Propylencarbonat, Sulfolan, 1,2,4-Trimethylbenzol oder Mesitylen, bevorzugt aber lösemittelfrei durchgeführt. Dazu werden die teilhydrierten oder perhydrierten, bevorzugt die N-Alkyltetrahydrocarbazole, mit Aktivkohle versetzt. Im Allgemeinen ist die Aktivkohle dampfbehandelt und/oder sauer gewaschen. Geeignete Aktivkohlen sind beispielsweise aus Torf hergestellte und anschließend mit Dampf aktivierte und noch sauer gewaschene Aktivkohlen, wie Norit SX2^{®}, sauer hergestellte und nur dampfbehandelte, sowie dampfbehandelte und sauer gewaschene Aktivkohlen aus Torf. Ebenso geeignet sind aus Braunkohle, Steinkohle, Holz, Nussschalen, Tierkohle, landwirtschaftlichen Reststoffen sowie aus Rückständen der Biogasproduktion hergestellte Aktivkohlen mit und ohne Nachbehandlung oder Aktivierung, wie sie für die aus Torf hergestellten Aktivkohlen beschrieben sind. Exemplarisch seien Norit SX2^{®}, Carbopal PA4^{®}, Carbopal MB4^{®} und Carbopal SC11 PG^{®} genannt. Die BET-Oberflächen der Aktivkohlen betragen beispielsweise 700 bis 1900 m²/g.

Bevorzugt sind aus Torf hergestellte Aktivkohlen.

Die Aktivkohle kann in Mengen von beispielsweise 5 bis 15% (m/m), bezogen auf die Menge des teilhydrierten oder perhydrierten N-Alkylcarbazols, eingesetzt werden.

In einer bevorzugten Ausführungsform der Erfindung wird die Aromatisierung in flüssiger Phase in Abwesenheit eines separaten Lösungsmittels durchgeführt wird. Der Verzicht auf ein separates Lösungsmittel ist unter Sicherheitsaspekten zusätzlich vorteilhaft.

In weiteren Ausführungsformen der Erfindung wird die Aromatisierung in einem separaten Lösungsmittel durchgeführt. Bevorzugte Lösungsmittel sind ausgewählt ist aus der Gruppe bestehend aus Diethylenglycol, Triethylenglycol, Diglyme, Triglyme, N-Methylpyrrolidon, Ethylencarbonat, Propylencarbonat, Sulfolan, 1,2,4-Trimethylbenzol und Mesitylen.

Die Aromatisierung wird vorzugsweise bei 180 bis 280°C, besonders bevorzugt bei 190 bis 260°C und insbesondere bei 200 bis 250°C durchgeführt. Die Reaktionszeit kann beispielsweise 12 - 72 Stunden, vorzugsweise 18 - 60 Stunden betragen. Im Allgemeinen erfolgt die Umsetzung bei einem Druck von 0,5 bis 5 bar, insbesondere bei Umgebungsdruck.

Die Aromatisierung wird unter einer sauerstoffhaltigen Gasatmosphäre mit einem Sauerstoffgehalt von 10 bis 30 Vol.-%, bevorzugt 15 bis 25 Vol.-%, besonders bevorzugt unter einer Luftatmosphäre durchgeführt. Zum Abtransport des gebildeten Wasserstoffs wird im Allgemeinen ein leichter sauerstoffhaltiger Gasstrom der oben genannten Zusammensetzung, bevorzugt ein leichter Luftstrom, über oder durch das Reaktionsgemisch geleitet.

In einer bevorzugten Ausführungsform der wird also ein Strom der sauerstoffhaltigen Atmosphäre, bevorzugt ein Luftstrom, über oder durch das Reaktionsgemisch, enthaltend das teilhydrierte oder perhydrierte N-Alkylcarbazol und die Aktivkohle, geleitet.

Nach erfolgtem Umsatz (feststellbar beispielsweise durch Kontrolle via DC oder < 5 % (a/a) Edukt im GC bzw. HPLC) kann die Aktivkohle heiß vom Produkt und ggf. dem Lösungsmittel filtriert werden. Das Produkt fällt so bereits sehr sauber an. Überraschenderweise gelingt die Aromatisierung also auch ohne das Vorhandensein von (Edel)metallen. Im Falle einer lösungsmittelfreien Fahrweise kann durch Umkristallisieren des erhaltenen aktivkohlefreien Filtrats, beispielsweise im Falle von N-Ethylcarbazol aus Methanol, ein analysenreines Produkt erhalten werden. Selbstverständlich kann auch die am Ende der Umsetzung erhaltene Reaktionsmischung nach Erkalten aus einem geeigneten Lösungsmittel (im Falle von N-Ethylcarbazol vorzugsweise Methanol) umkristallisiert werden.

Wird die Aromatisierung in Gegenwart eines Lösungsmittels durchgeführt, kann das Rohprodukt durch Zugabe eines geeigneten Lösungsmittels ausgefällt werden und bei Bedarf z.B. durch Umkristallisation oder Destillation weiter aufgereinigt werden.

Eine Vorreinigung der Edukte ist, im Unterschied zur Palladium-katalysierten Aromatisierung, nicht erforderlich. Beim Einsatz von Edukten geringerer Reinheit wird unter Umständen eine größere Menge an Aktivkohle sowie eine längere Reaktionszeit zum vollständigen Umsatz benötigt.

Eine Vorreinigung der N-Alkyl-1,2,3,4-tetrahydrocarbazole kann beispielsweise durch halbstündiges Rühren in etwa 60°C warmem Toluol mit 1-2 % (m/m) Aktivkohle bewerkstelligt werden. Nach Filtration und Entfernen des Lösungsmittels wird reineres N-Alkyl-1,2,3,4-tetrahydrocarbazol erhalten, das mit weniger Aktivkohle schneller als ungereinigtes Startmaterial aromatisiert werden kann. Die Vorreinigung kann z.B. beim N-Ethyl-1,2,3,4-tetrahydrocarbazol auch durch Vakuumdestillation erfolgen.

Die Erfindung wird durch die nachstehenden Beispiele näher erläutert.

### Beispiele

### Vergleichsbeispiele 1 bis 3

### Aromatisierung unter Stickstoffatmosphäre mit verschiedenen Aktivkohle-Typen

### Vergleichsbeispiel 1

In einem 50 ml-Vierhalsrundkolben mit Magnetrührer und innen verbautem Temperaturfühler sowie Kühler und Stickstoffanschluss wurden 5 g N-Ethyltetrahydrocarbazol und 0,5 g Aktivkohle vom Typ I (Donau Carbon Carbopal PA4 mit einer BET-Oberfläche von ca. 1700 m²/g) vorgelegt, inertisiert und unter Rühren auf 250 °C Innentemperatur geheizt. Eine Reaktionskontrolle erfolgte durch Dünnschichtchromatographie auf Kieselgelplatten mit einer 5:1 Mischung aus n-Heptan und Ethylacetat nach 3 h, 6 h, 9 h und 12 h. Eine weitere Auswertung erfolgte durch HPLC. Die Ergebnisse sind Tabelle 1 wiedergegeben.

Zur Aufarbeitung wurde die Aktivkohle in einem beheizbaren Druckfilter mit leichtem Überdruck abgetrennt. Es wurde ohne Lösungsmitteleinsatz gearbeitet.

### Vergleichsbeispiel 2

Durchführung analog zu Beispiel 1, jedoch mit Aktivkohle vom Typ II (Donau Carbon Carbopal MB4 mit einer BET-Oberfläche von ca. 900 m²/g). Die Ergebnisse sind in Tabelle 1 wiedergegeben.

### Vergleichsbeispiel 3

Durchführung analog zu Beispiel 1, jedoch mit Aktivkohle vom Typ III (Donau Carbon Carbopal SC11 PG mit einer BET-Oberfläche von ca. 950 m²/g). Die Ergebnisse sind in Tabelle 1 wiedergegeben.

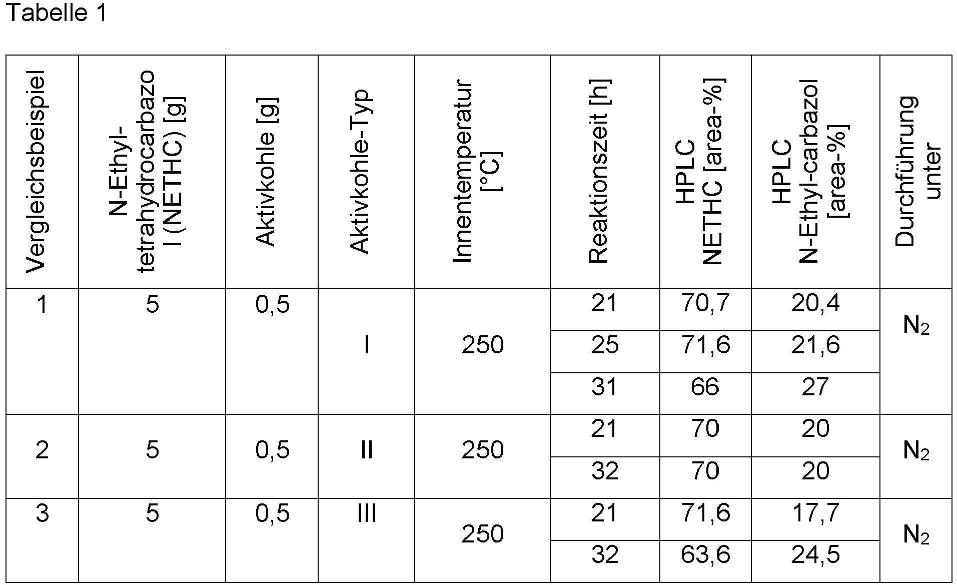

Wie die Vergleichsbeispiele 1 bis 3 zeigen, gibt es einen Unterschied in der Reaktivität zwischen den Aktivkohletypen. Trotz hoher Reaktionstemperatur von 250 °C wurden jedoch nur geringe Umsätze unter N₂-Atomphäre erreicht. Ein Lösungsmittel wurde nicht verwendet.

### Erfindungsgemäße Beispiele unter Luftatmosphäre mit verschiedenen Aktivkohle-Typen

### Beispiele 1 bis 5

### Beispiel 1

In einem 50 ml-Vierhalsrundkolben mit Magnetrührer und innen verbautem Temperaturfühler sowie Kühler und Druckluftanschluss wurden 5 g N-Ethyltetrahydrocarbazol (NETHC) und 0,5 g Aktivkohle vom Typ IV (Norit SX2) vorgelegt und unter Rühren auf 230°C Innentemperatur geheizt. Während der gesamten Reaktionszeit lag ein leichter Luftstrom an. Eine Reaktionskontrolle erfolgte durch Dünnschichtchromatographie auf Kieselgelplatten mit einer 5:1 Mischung aus n-Heptan und Ethylacetat sowie durch HPLC. Die Ergebnisse sind in Tabelle 2 wiedergegeben.

Zur Aufarbeitung wurde die Aktivkohle in einem beheizbaren Druckfilter mit leichtem Überdruck abgetrennt. Es wurde ohne Lösungsmittel gearbeitet.

### Beispiel 2

Durchführung analog zu Beispiel 1, jedoch bei 190 °C Reaktionstemperatur. Die Ergebnisse sind in Tabelle 2 wiedergegeben.

### Beispiel 3

Durchführung analog zu Beispiel 1, jedoch bei 180 °C Reaktionstemperatur. Die Ergebnisse sind in Tabelle 2 wiedergegeben.

### Beispiel 4

Durchführung analog zu Beispiel 1, jedoch mit Aktivkohle vom Typ III. Die Ergebnisse sind in Tabelle 2 wiedergegeben.

### Beispiel 5

Durchführung analog zu Beispiel 1, jedoch mit reduzierter Aktivkohlemenge (0,2 g). Die Ergebnisse sind in Tabelle 2 wiedergegeben.

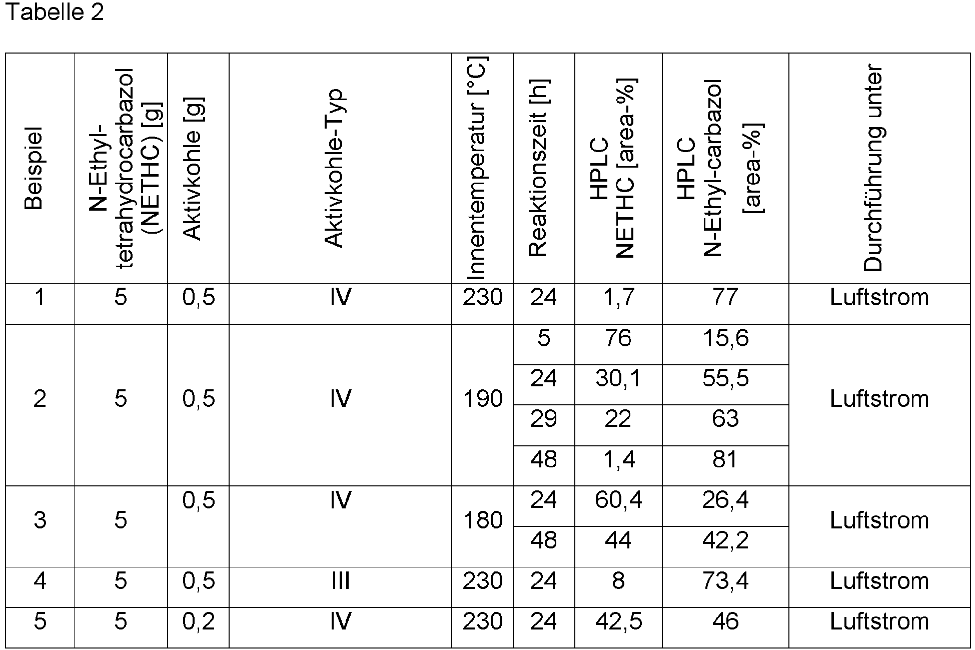

## Patentansprüche

1. Verfahren zur Herstellung von N-Alkylcarbazolen der allgemeinen Formel 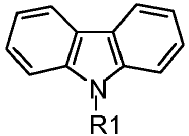
worin R₁ ein linearer oder verzweigter C₁-C₁₂-Alkylrest ist,
durch metallfreie Aromatisierung eines teilhydrierten oder perhydrierten N-Alkylcarbazols in Gegenwart von Aktivkohle und Sauerstoff, **dadurch gekennzeichnet, dass** das teilhydrierte oder perhydrierte N-Alkylcarbazol in Gegenwart von Aktivkohle unter einer sauerstoffhaltigen Atmosphäre mit einem Sauerstoffgehalt von 10 bis 30 Vol.-% auf eine Temperatur von 150 bis 300°C erhitzt wird.

2. Verfahren nach Anspruch 1, **dadurch gekennzeichnet, dass** ein teilhydriertes N-Alkylcarbazol aromatisiert wird.

3. Verfahren nach Anspruch 2, **dadurch gekennzeichnet, dass** ein N-Alkyl-1,2,3,4-tetrahydrocarbazol der allgemeinen Formel 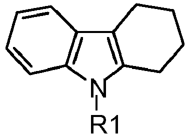 aromatisiert wird.

4. Verfahren nach Anspruch 3, **dadurch gekennzeichnet, dass** das N-Alkyl-1 ,2,3,4-tetrahydrocarbazol durch Fischer-Indolsynthese aus Cyclohexanon und dem entsprechenden N-Alkyl-N-Phenylhydrazin hergestellt wird.

5. Verfahren nach einem der Ansprüche 1 bis 4, **dadurch gekennzeichnet, dass** R₁ ein linearer oder verzweigter C₁-C₈-Alkylrest ist.

6. Verfahren nach Anspruch 5, **dadurch gekennzeichnet, dass** R₁ Ethyl ist.

7. Verfahren nach einem der Ansprüche 1 bis 6, **dadurch gekennzeichnet, dass** die Aromatisierung in Abwesenheit eines separaten Lösungsmittels durchgeführt wird.

8. Verfahren nach einem der Ansprüche 1 bis 7, **dadurch gekennzeichnet, dass** die sauerstoffhaltige Atmosphäre eine Luftatmosphäre ist.

9. Verfahren nach einem der Ansprüche 1 bis 8, **dadurch gekennzeichnet, dass** ein Strom der sauerstoffhaltigen Atmosphäre über oder durch das Reaktionsgemisch, enthaltend das teilhydrierte oder perhydrierte N-Alkylcarbazol und die Aktivkohle, geleitet wird.

10. Verfahren nach einem der Ansprüche 1 bis 9, **dadurch gekennzeichnet, dass** zur Aromatisierung auf eine Temperatur von 190 bis 260°C erhitzt wird.

11. Verfahren nach einem der Ansprüche 1 bis 10, **dadurch gekennzeichnet, dass** die Aktivkohle aus Torf hergestellt wird.

12. Verfahren nach einem der Ansprüche 1 bis 11, **dadurch gekennzeichnet, dass** die Aktivkohle dampfbehandelt und/oder sauer gewaschen ist.

13. Verfahren nach einem der Ansprüche 1 bis 12, **dadurch gekennzeichnet, dass** die BET-Oberfläche der Aktivkohle 700 bis 1900 m²/g beträgt.
